# EUROPEAN PATENT APPLICATION

(11) **EP 4 737 893 A1**
(43) Date of publication of application: **06.05.2026**
(21) Application number: 25209068.3
(22) Date of filing: 16.10.2025
(51) Int. Cl.: G01N 27/407, G01N 33/00

(54) **PROTON CONDUCTOR GAS SENSOR**

(30) Priority: 31.10.2024 JP 2024191496
(71) Applicant: Figaro Engineering Inc., Mino, Osaka 562-8505 (JP)
(72) Inventor: SUGITA, Norio, Mino, Osaka, 562-8505 (JP)
(74) Representative: Kuhnen & Wacker Patent- und Rechtsanwaltsbüro PartG mbB

(57) **Abstract**

A proton conductor gas sensor (2) comprises: an MEA (20); a diffusion control plate (6); a filter (12); and a housing (4). The diffusion control plate (6) is provided with a protrusion (22) with a diffusion control hole (24) at the center and raising towards the opposite side to the MEA (20). A gap (26) is present between the protrusion (22) and the MEA (20). The output variance among proton conductor gas sensors is reduced by the protrusion.

## Description

### Field of the Invention

The present invention relates to a proton conductor gas sensor and particularly, a diffusion control plate that controls the diffusion of atmosphere from a sealing cap to a membrane electrode assembly (MEA) of the gas sensor.

### Background Art

The applicant has proposed a proton conductor gas sensor that comprises an MEA, a sealing cap, and a diffusion control plate (Patent Document 1: USP 7393505B). The sealing cap is a container with two metal plates that accommodates a filter comprising activated charcoal. It introduces atmosphere through its side or top surface, processes the atmosphere through the filter, and supplies the atmosphere through an opening towards the MEA. Furthermore, the sealing cap functions as an output terminal connected to the detection electrode of the gas sensor. The diffusion control plate, which supplies atmosphere through its diffusion control hole, is present between the opening of the sealing cap and the MEA. The MEA contains a proton conductor membrane at its center, as well as catalytic electrodes on both sides of the membrane, and gas diffusion layers on and outside of both electrodes. The catalytic electrodes contain a carbon that supports electrode catalyst, such as platinum (Pt), and a proton conductor, such as Nafion (a registered trademark of the DuPont Company). The gas diffusion layers contain carbon, such as carbon black or activated charcoal. They are porous to allow gas diffusion and provide electrical conductivity. To control the hydrophobicity and hydrophilicity of the gas diffusion layers, Teflon (a registered trademark of DuPont) and Nafion are added to the layers.

The output of this gas sensor is proportional to the concentration of the gas to be detected in the atmosphere. This is achieved by controlling the quantity of atmosphere provided to the MEA with the gas diffusion hole of a controlled size. The size of the hole is made accurate by creating it in a thin diffusion control plate, since a thin plate allows to accurate machining.

### Prior Document List

### Patent Document

Patent Document 1: USP 7393505B

### Summary of the Invention:

### Object of the Invention:

The inventor has found that the material of the MEA (e.g., one of the gas diffusion layers) sometimes enters the diffusion control hole of the diffusion control plate. This causes variance in the diffusion of the atmosphere in the gas diffusion hole. This variance results in output variance between gas sensors.

The object of the invention is to prevent the gas diffusion layer material from entering the diffusion control hole of the diffusion control plate of the proton conductor gas sensor and reduce output variance between proton conductor gas sensors.

### Means for Solving the Problem

The proton conductor gas sensor according to the invention comprises:
an MEA having a proton conductor membrane; catalytic electrodes on both sides of the proton conductor membrane; and a pair of gas diffusion layers on the catalytic electrodes in an opposite side to the proton conductor membrane;
a diffusion control plate in contact with one face of the MEA, pressing the MEA , and having a diffusion control hole supplying atmosphere to the MEA;
a filter supplying atmosphere to the diffusion control plate; and
a housing accommodating the MEA; the diffusion control plate; and the filter.
The gas sensor is characterized in
that the diffusion control plate is provided with a protrusion raising towards the opposite side to the MEA wherein the diffusion control hole is at a center portion of the protrusion, and
that a gap is present between the protrusion and the MEA.

Preferably, the gas sensor further comprises: a sealing cap fixed within the housing; accommodating the filter; and having an atmosphere introducing hole at the opposite side to the diffusion control plate seen from the filter, and an atmosphere supplying hole at a side facing the diffusion control plate; and a gasket pressing the sealing cap towards the diffusion control plate and making the sealing cap in contact with the diffusion control plate.

The protrusion is accommodated in the atmosphere supplying hole, and
the atmosphere supplying hole is larger in diameter than the protrusion of the diffusion control hole.

Preferably, the atmosphere supplying hole of the sealing cap is larger in thickness than the height of the protrusion, and a clearance is present between the protrusion and the filter. This prevents the contact between the protrusion of the diffusion control plate and the filter, even when the height of the protrusion is excessively high, and reduces the variance in the gas sensitivity. Preferably, the diffusion control plate is made of a metal.

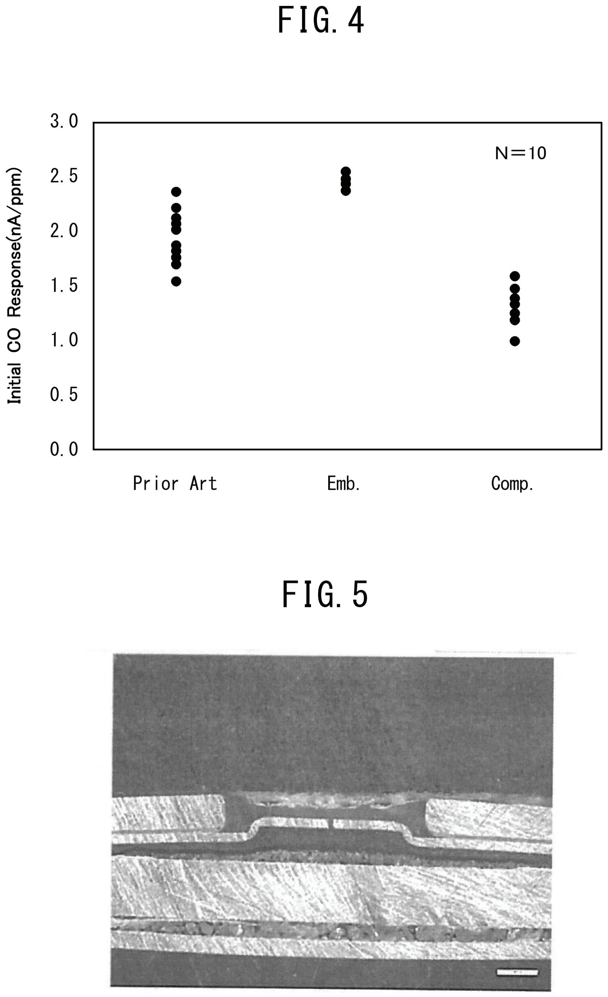

According to the invention, the pressure that the diffusion control plate compresses the MEA secures the electrical connection between the gas diffusion layer of the MEA and the diffusion control plate. Due to the pressure, the material of the gas diffusion layer enters sometimes the diffusion control hole of the diffusion control plate. However, according to the invention, the diffusion control plate is provided with the protrusion protruding to the opposite direction to the MEA, and the diffusion control hole is present at the center of the protrusion. Therefore, a gap is present between the protrusion and the MEA, and the material for the gas diffusion layer does not enter the diffusion control hole. As a result, the variance in the gas sensor output per gas concentration is reduced (cf. the comparative example and embodiment in Fig. 4).

Preferably, the sealing cap is provided with a ring-like protrusion around the atmosphere supplying hole protruding in an opposite side to the diffusion control plate, and the atmosphere supplying hole of the sealing cap is made larger in thickness by the ring-like protrusion. When the thickness of the sealing cap is increased for increasing the thickness of the atmosphere supplying hole, the machining of the sealing cap becomes difficult. In contrast, when a ring-like protrusion protruding towards the other side to the diffusion control plate is provided, the thickness of the atmosphere supplying hole is simply increased. The ring-like protrusion is simply made by burring machining or welding a ring.

Preferably, the filter is provided with a recess at a portion facing the atmosphere supplying hole. The recess prevents the contact between the protrusion and the filter, even when the protrusion of the diffusion control plate is excessively high, and reduces the variance in the gas sensitivity.

### Brief Description of the Drawings

Fig. 1: A sectional view of a proton conductor gas sensor according to an embodiment.
Fig. 2: A partially enlarged sectional view of a gas diffusion plate and MEA according to the embodiment.
Fig. 3: A partially enlarged sectional view of a gas diffusion plate and MEA of a conventional gas sensor.
Fig. 4: A characteristic diagram showing the distributions of CO sensitivities for the embodiment, the conventional gas sensor, and a comparative example.
Fig. 5: A microscopic image of a partial section of the gas sensor according to the embodiment, indicating the gap between the MEA and the diffusion control plate.
Fig. 6: A microscopic image of a partial section of a conventional gas sensor indicating the gas diffusion layer material that entered the diffusion control hole.
Fig. 7: A sectional view of a proton conductor gas sensor according to a modification.
Fig. 8: A sectional view of a proton conductor gas sensor according to a second modification.
Fig. 9: A sectional view of a proton conductor gas sensor according to a third modification.
Fig. 10: A partially enlarged sectional view of a proton conductor gas sensor according to a second embodiment.
Fig. 11: A partially enlarged plan view of the sealing cap and diffusion control plate according to the second embodiment.
Fig. 12: A partially enlarged sectional view of a proton conductor gas sensor according to a third embodiment.

### Means for carrying out the invention:

The best embodiment for carrying out the invention will be described.

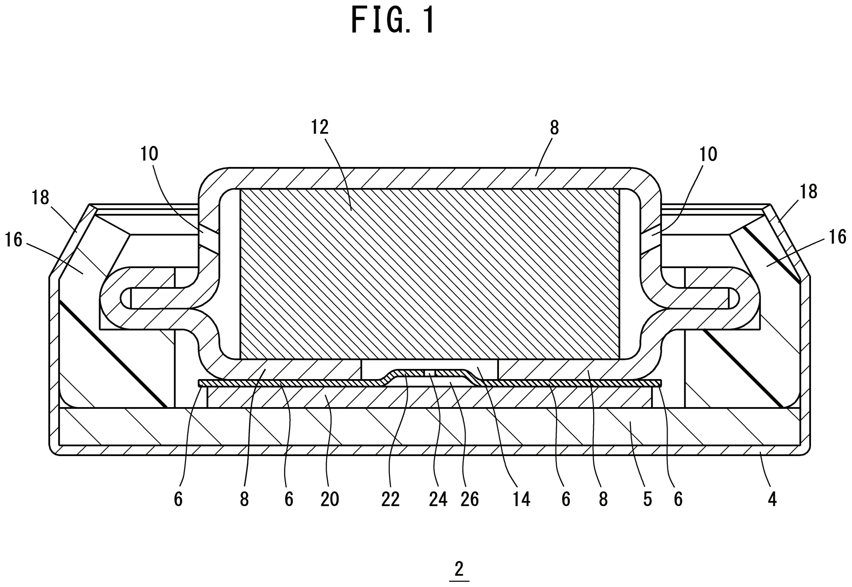

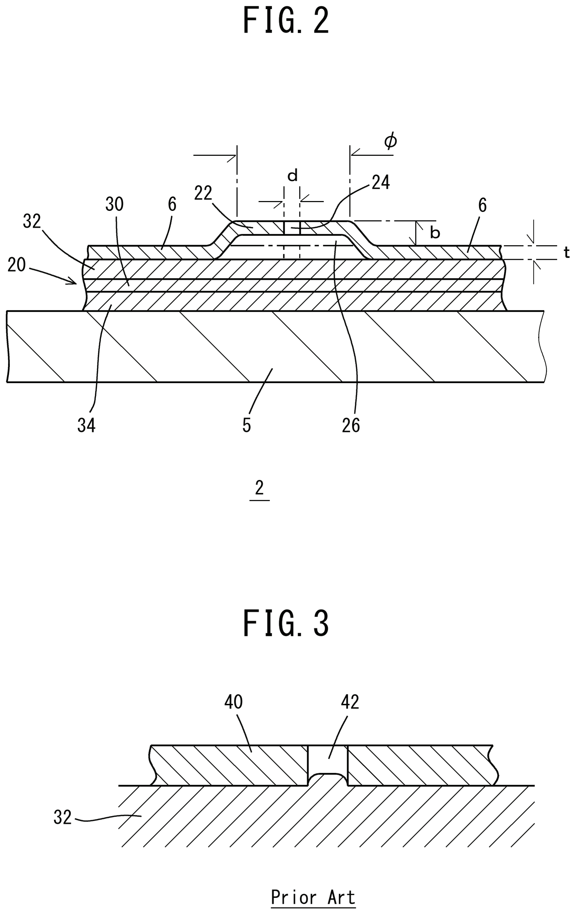

Fig. 1 shows a proton conductor gas sensor 2 according to one embodiment, and Fig. 2 shows its major portions. The metal can 4 has a bottom plate 5 on its bottom surface and a side wall 18 on its outer periphery. The bottom plate 5 may be absent. An MEA is indicated by 20; its bottom surface is in contact with the bottom plate 5 and its top surface is in contact with a diffusion control plate 6 made of metal. The diffusion control plate's top surface is in contact with the sealing cap's bottom surface. The sealing cap 8 is a circular, disc-shaped container that comprises two metal plates and accommodates, in its inner space, a filter 12, such as activated charcoal, silica gel, zeolite, or alumina. It has an atmosphere-introducing hole 10 on its top or side face and an atmosphere supplying hole 14 on its bottom. A ring-like gasket 16 presses the sealing cap 8 towards the diffusion control plate 6, and the electrical connection is maintained by the pressure between the sealing cap 8, the diffusion control plate 6, the MEA 20, the bottom plate 5, and the can 4.

The diffusion control plate 6 has a disc-like protrusion 22 with one diffusion control hole 24 at its center, for example. The protrusion has a smaller diameter than the atmosphere supplying hole 14 and is accommodated inside it. A gap 26 is present between the protrusion 22 and the MEA 20. A plurality of diffusion control holes 24 may be provided on the protrusion 22.

Fig. 2 shows the diffusion control plate 6 and the MEA 20. The MEA 20 is a five-layer MEA comprising a proton conductor membrane 30 with catalytic electrodes on its front and rear surfaces, as well as two gas diffusion layers 32, 34 in contact with the catalytic electrodes. The proton conductor membrane is made of a polymer, such as Nafion (a registered trademark of the DuPont Company), and the catalytic electrodes are made of electrically conductive carbon, such as carbon black, graphite, and activated charcoal, which supports an electrode catalyst, such as platinum (Pt). The gas diffusion layers are made of electrically conductive carbon, such as carbon black, graphite, activated charcoal, and carbon fiber, and are formed into layers with a binder, such as Teflon. The gas diffusion layers diffuse the atmosphere, supply the proton conductor membrane with the necessary water, evacuate excess water from the membrane, and form electrical connection paths between the catalytic electrodes and the sealing cap 8, as well as between the catalytic electrodes and the bottom plate 5. The catalytic electrodes facing the diffusion control plate 6 are called detection electrodes, and the electrode facing the bottom plate 5 is called the counter electrode.

Exemplar sizes related to the diffusion control plate 6 are indicated below. For example, the diameter of the atmosphere supplying hole 14 is 2.5 mm, the outer diameter (*ϕ*) of the disc-like protrusion 22 is 1.5 mm, the diameter (d) of the diffusion-control hole 24 is 0.1 mm, the height (b) of the protrusion 22 is 0.2 mm, and the thickness (t) of the diffusion-control plate 6 is 0.1 mm. The gas diffusion layers 32 and 34 are generally 100 to 1000 micro-meter thick, and the exemplar thickness is 200 micro-meters. The quantity of atmosphere supplied to the MEA 20 is proportional to the gas flow rate in the gas diffusion control hole 24. The gas sensor 2 output is also proportional to the gas flow rate in the gas diffusion control hole 24. The various sizes themselves related to the protrusion 22 are arbitrary.

Fig. 3 shows the major portions of a conventional gas sensor. A planar diffusion control plate 40 of the same size as the embodiment, without the disc-like protrusion, is arranged on the gas diffusion layer 32, where a diffusion control hole 42 of the same size as the embodiment is provided.

The inventor has found that the material of the gas diffusion layer 32 sometimes enters the diffusion control hole 42. This situation is depicted schematically in Fig. 3. The inventor speculates that, since the gas diffusion layer comprises plastic carbon powder or a plastic carbon sheet, a portion of the material entered the diffusion control hole by the pressure of the diffusion control plate.

Ten pieces each of conventional gas sensors (Fig. 3), gas sensors according to the embodiment in Figs. 1 and 2, and comparative gas sensors with a diffusion control plate facing in the opposite direction from that in Figs. 1 and 2 were prepared. In the comparative example, the disc-like protrusion 22 faced the gas diffusion layer 32. The gas sensors were made in contact with CO at room temperature, and the output currents per 1 ppm of CO in the atmosphere were measured. Fig. 4 shows the distributions of the output currents. According to the embodiment, the mean output current was high, and the variance in output currents was low. This indicates that the material of the gas diffusion layer 32 did not enter the diffusion control hole 24, thereby allowing a large quantity of atmosphere to be supplied to the MEA 20 with a small variance in the gas flow rate in the diffusion control hole 24. In contrast, the mean outputs of the gas sensors in the conventional and comparative examples were small, and the outputs had a large variance. This suggests that the gas diffusion layer material entered the diffusion control hole, thereby fluctuating the gas flow rate.

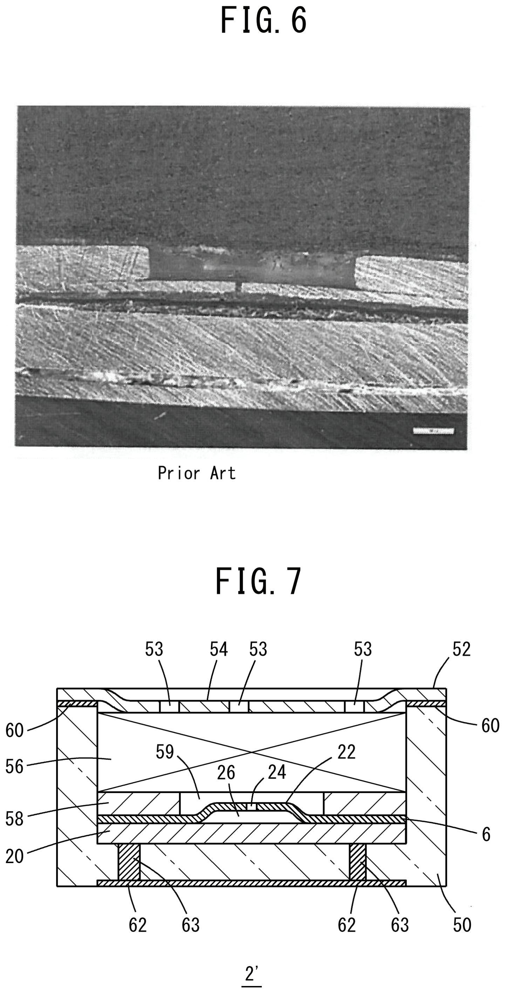

Fig. 5 is a microscopic image of the major portions of the gas sensor 2 according to the embodiment, and Fig. 6 is a microscopic image of the major portions of the conventional gas sensor 2. In both the embodiment and the conventional example, the MEAs expanded at the portions of the atmosphere supplying hole due to counteraction by the pressure from the sealing cap. Therefore, the pressure pushes the gas diffusion layer material into the diffusion control hole. However, in the embodiment, a disc-like protrusion is present at the center of the atmosphere supplying hole in the sealing cap. The protrusion generates a gap between itself and the MEA. Thus, the material of the gas diffusion layer does not enter the diffusion control hole.

### Modifications

Fig. 7 shows a proton conductor gas sensor 2' according to a first modification. It is similar to the embodiment in Figs. 1 and 2, except for those particularly specified. The housing 50, which is made of ceramic, has a metal lid 52 that is fixed to the housing. The lid 52 has an opening 53 and a disc-like protrusion 54. The lid 52 also works as a terminal for external electrical connection and as a leaf spring. Shaped activated charcoal or similar material is used for the filter 56. The activated charcoal in the filter is electrically conductive. When an insulating filter material, such as silica gel, alumina, or zeolite, is used, electrical conductivity is provided to the filter material by dispersing graphite or other materials into the filter material. A metal plate 58 has an opening 59 in its center. Indicated by 60-63 are metallized portions in the housing 50.

The MEA 20 and the diffusion control plate 6 are interposed between the upper bottom surface of the housing 50 and metal plate 58. The elastic force of lid 52 creates sequential contact between the lid 52, the filter 56, the diffusion control plate 6, the MEA 20, and the housing 50. Both electrical paths, one from the MEA 20 to the lid 52 and the other from the MEA 20 to the metallized portion 62, are secured. Furthermore, the metallized portion 62 are made electrically conductive to fix the lid 52 to the housing 50 by welding or similar methods. Alternatively, the lid 52 can be fixed to the housing 50 using an adhesive.

According to this modification, the gap 26 prevents the gas diffusion layer material from entering the diffusion control hole 24. As a result, the quantity of supplied atmosphere to the MEA 20 is uniform among the gas sensors, and this makes the gas sensor output per unit gas concentration also uniform.

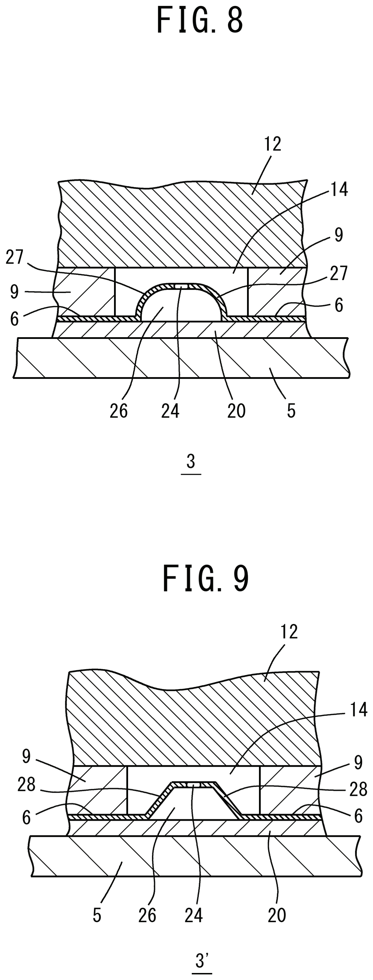

Fig. 8 shows a proton conductor gas sensor 3 according to a second modification. It is similar to the embodiment in Figs. 1 and 2, except for those particulalrly specified. The diffusion control plate 6 has a hemi-spherical protrusion 27 with a diffusion control hole 24 at the top. Since the height of protrusion 27 is greater than that of protrusion 22, a sealing cap 9 with greater thickness at the bottom than in Fig. 1 is used. The protrusion 27 is accommodated within the atmosphere supplying hole 14.

Fig. 9 shows a third modification of the proton conductor gas sensor 3', which is similar to the sensor in Fig. 8. The diffusion control plate 6 has a trapezoidal protrusion 28 with a flat top surface that is parallel to its bottom face, and the flat top surface of the protrusion 28 has the diffusion control hole 24.

The proton conductor gas sensor 2 may be provided with an additional hole in the bottom plate 5 to allow water vapor from a water reservoir below the bottom plate 5 to reach the MEA 20.

### Second and Third Embodiments

The inventor has found that when the height b of the protrusion 22 in the diffusion control plate 6 is excessively high, the top of the protrusion 22 sometimes touches the filter 12. As a result, the variance in the gas sensor 2 outputs increases with smaller CO sensitivities. Therefore, gas sensors without the protrusion (the conventional example) and gas sensors according to the embodiment with protrusions of heights 0.2 and 0.3 mm were prepared to measure CO sensitivities and variances. Measurements were performed with and without the filter (Table 1 and Table 2, respectively).

The atmosphere supplying hole 14 had a diameter of 2.5 mm, the protrusion 22 had an outer diameter of 1.5 mm, the diffusion control hole 24 had a diameter of 0.1 mm, and the diffusion control plate 6 had a thickness of 0.1 mm. The sealing cap 8 had a thickness of 0.4 mm at the portion facing the diffusion control plate 6. The filter 12 was a shaped body of powder-like activated charcoal with a synthetic resin binder, coated with non-woven cloth on its upper and lower surfaces to prevent scattered activated charcoal powder. "N" in the tables indicates the number of samples.

**TABLE 1 (with filter)**

| Sample: Conventional Example (N = 20), Embodiment (N = 10), Embodiment (N = 10) (without protrusion) (Protrusion Height: 0.2 mm) (Protrusion Height: 0.3 mm) | | | |
|---|---|---|---|
| CO sensitivity: | 1.92 | 2.50 | 2.08 |
| (nA/ppm) | | | |
| Standard Deviation: | 0.20 | 0.05 | 0.19 |

**TABLE 2 (without Filter)**

| Sample: Conventional Example (N = 20), Embodiment (N = 10), Embodiment (N = 10) (without Protrusion) (Protrusion Height: 0.2 mm) (Protrusion Height: 0.3 mm) | | | |
|---|---|---|---|
| CO sensitivity: | 2.14 | 2.72 | 2.88 |
| (nA/ppm) | | | |
| Standard Deviation: | 0.16 | 0.05 | 0.05 |

As shown in Table 1, increasing the height of protrusion 22, the raised height of diffusion control plate 6 in the vicinity of diffusion control hole, to 0.3 mm for a gas sensor with filter 12 increased variance in CO sensitivity, output current from gas sensor, and decreased CO sensitivity.

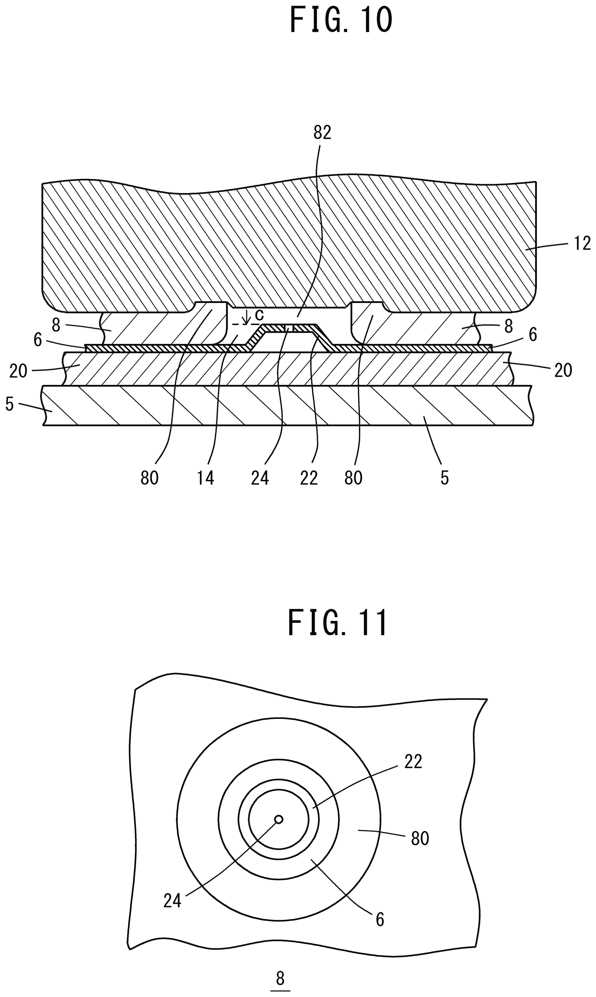

Table 2 shows the CO sensitivity and its variance for gas sensors without the filter. Regardless of whether the protrusion 22 was 0.3 mm or 0.2 mm, the variance in CO sensitivities and also the CO sensitivities were the same. This indicates that when the protrusion is excessively high, the diffusion control plate is in contact with the filter, restricting the diffusion of atmosphere into the diffusion control hole. Therefore, preventing the filter 12 from entering the diffusion control hole 14, as shown in Figs. 10 and 11, reduced the variance in CO sensitivities, when the protrusion 22 was high.

To prevent the diffusion control plate 6 from contacting the filter 12 at the protrusion 22, the bottom portion of the sealing cap 8 can be thickened. However, this complicates the machining of the sealing cap 8. Therefore, it is preferable to make a ring-like protrusion around the atmosphere supplying hole 14 on the sealing cap 8 instead of increasing its bottom portion's thickness. To make the ring-like protrusion, burring machining is applied to the sealing cap when the atmosphere supplying hole is made. Burring machining is a type of punching machining in which a ring-like protrusion is formed around a hole by extruding the material from the hole during punching. Alternatively, a ring-like member that encloses the atmosphere supplying hole 14 can be attached to the sealing cap 8 on the surface opposite the diffusion control plate 6. Additionally, the top surface of the protrusion may not be flat. Such an embodiment is shown in Figs. 10 and 11. The ring-like protrusion around the atmosphere supplying hole 14 is indicated by 80 and is made by burring machining. The filter 12 is compressed by the protrusion 80, a recess 82 is formed. The clearance between the top face of the protrusion 22 and the filter 12 is designated c. Regarding other points, this embodiment is the same as that in Figs. 1 and 2.

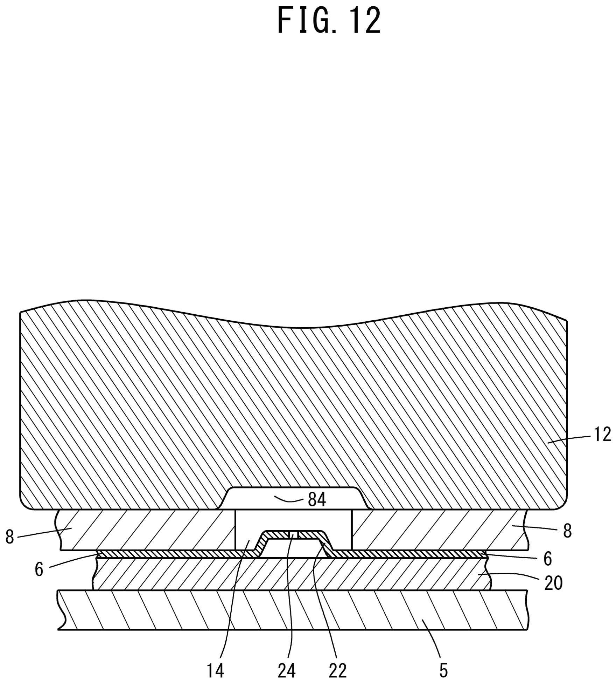

Fig. 12 shows an alternative embodiment in which a recess 84 is formed in the filter 12, instead of a protrusion on the sealing cap 8. When shaping the filter, the recess is provided at a portion facing the atmosphere supplying hole. The filter is formed by mixing powdered adsorbent and a binder, then molding the filter with the recess, using the mixture with a die that corresponds to the shape of the recess. Alternatively, the recess can be formed by machining an adsorbent that has been shaped into a chip.

According to the embodiments, the variance in CO sensitivities is reduced. Additionally, the CO sensitivity is increased. The gas to be detected may be hydrogen, ethanol, etc., and is not limited to CO.

List of Symbols
- 2,2',3,3':: proton conductor gas sensor
- 4:: can
- 5:: bottom plate
- 6:: diffusion control plate
- 8,9:: sealing cap
- 10:: atmosphere introducing hole
- 12:: filter
- 14:: atmosphere supplying hole
- 16:: gasket
- 18:: side wall
- 20:: MEA
- 22:: disc-like protrusion
- 24:: diffusion control hole
- 26:: gap
- 27:: hemi-spherical protrusion
- 28:: trapezoidal protrusion in side view.
- 30:: proton conductor membrane with electrodes
- 32, 34:: gas diffusion layer

- 40:: (conventional) diffusion control plate
- 42:: (conventional) diffusion control hole

- 50:: housing
- 52:: lid
- 53:: opening
- 54:: protrusion
- 56:: filter
- 58:: metal plate
- 59:: opening
- 60-63:: metallized portion

- 80:: ring-like protrusion
- 82, 84:: recess (in filter)

- *ϕ* :: outer diameter of the protrusion
- b:: height of protrusion
- c:: clearance

## Claims

1. A proton conductor gas sensor (2,2',3,3') comprising: an MEA (20) having a proton conductor membrane (30); catalytic electrodes on both sides of the proton conductor membrane (30); and a pair of gas diffusion layers (32,34) on the catalytic electrodes in an opposite side to the proton conductor membrane (30);
a diffusion control plate (6) in contact with one face of the MEA (20), pressing the MEA (20), and having a diffusion control hole (24) supplying atmosphere to the MEA (20);
a filter (12) supplying atmosphere to the diffusion control plate (6); and
a housing (4) accommodating the MEA (20); the diffusion control plate (6); and the filter (12), **characterized in**
**that** said diffusion control plate (6) is provided with a protrusion (22, 27, 28) raising towards the opposite side to the MEA (20) wherein the diffusion control hole (24) is at a center portion of the protrusion (22, 27, 28), and
**that** a gap (26) is present between the protrusion (22, 27, 28) and the MEA (20).

2. The proton conductor gas sensor according to claim 1, **characterized in that** the gas sensor (2,3,3') further comprises: a sealing cap (8) fixed within the housing (4); accommodating said filter (12); and having an atmosphere introducing hole (10) at the opposite side to the diffusion control plate (6) seen from the filter (12), and an atmosphere supplying hole (14) at a side facing the diffusion control plate (6); and a gasket (16) pressing the sealing cap (8) towards the diffusion control plate (6) and making the sealing cap (8) in contact with the diffusion control plate (6),
that said protrusion (22,27,28) is accommodated in the atmosphere supplying hole (14), and
that said atmosphere supplying hole (14) is larger in diameter than the protrusion (22) of the diffusion control hole (6).

3. The proton conductor gas sensor according to claim 2, **characterized in that** the atmosphere supplying hole (14) of the sealing cap (8) is larger in thickness than the height of said protrusion (22,27,28), and
that a clearance is present between said protrusion (22,27,28) and said filter (12).

4. The proton conductor gas sensor according to claim 3, **characterized in that** said sealing cap (8) is provided with a ring-like protrusion (80) around said atmosphere supplying hole (14) protruding in an opposite side to said diffusion control plate (6), and
that the atmosphere supplying hole (14) of the sealing cap (8) is made larger in thickness by said ring-like protrusion (80).

5. The proton conductor gas sensor according to claim 2, **characterized in that** said filter (12) is provided with a recess (84) at a portion facing said atmosphere supplying hole (14).
